## Europäisches Patentamt
## European Patent Office
## Office européen des brevets

(19)

(11) Numéro de publication : **0 115 974**
**B1**

(12)

# FASCICULE DE BREVET EUROPÉEN

(45) Date de publication du fascicule du brevet :
07.09.88

(21) Numéro de dépôt : 84400019.0

(22) Date de dépôt : 05.01.84

(51) Int. Cl.⁴ : **A 61 K 37/54, C 12 N 9/96//**
**C12N9/52 ,(A61K37/54,**
**37:18)**

(54) Préparation à activité collagénolytique ayant une activité élevée et compositions pharmaceutiques la contenant.

(30) Priorité : 05.01.83 FR 8300114
05.01.83 FR 8300112

(43) Date de publication de la demande :
15.08.84 Bulletin 84/33

(45) Mention de la délivrance du brevet :
07.09.88 Bulletin 88/36

(84) Etats contractants désignés :
BE CH DE FR GB IT LI LU NL SE

(56) Documents cités :
GB-A- 802 069
US-A- 3 296 094
US-A- 3 677 900
BIOLOGICAL ABSTRACTS, vol. 70, no. 8, 1980,
résumé no. 53321, page 5603, Philadelphia (US) & J.
Bacteriol 142(2), 447-454, 1980 G.C. REID et al.:
"Peptone induction and rifampin-insensitive collagenase production by Vibrio alginolyticus"
BIOLOGICAL ABSTRACTS, vol. 73, no. 11, 1982,
résumé no. 78495, page 8191, Philadelphia (US) &
Arch. Microbiol., 130(4), 276-280, 1981 P. HARE et al.:
"Regulation of exoprotease production by temperature and oxygen in Vibrio alginolyticus".
MICROBIOLOGY ABSTRACTS, Section B, vol. 11, no.
3, mars 1976, (Information Retrieval, Londres G.B.),
pages 44, 45; résumé no. 11B2274 & FEBS Lett., 59(2),
167-172, 1975 A. LECROISEY et al.: "Purification,
stability and inhibition of the collagenase from Achromobacter iophagus".
MICROBIOLOGY ABSTRACTS, Section B, vol. 11, no.
3, mars 1976 (Information Retrieval, Londres G.B.),
page 44, résumé no. 11B2273 & FEBS Lett., 56(2), 292-
296, 1975 B. KEIL et al.: "Specificity of collagenase
from Achromobacter iophagus".

(73) Titulaire : INSTITUT PASTEUR
25-28, rue du Docteur Roux
F-75724 Paris Cédex 15 (FR)

(72) Inventeur : Hurion, Nicole
11 bis, Avenue Faraday
F-75017 Paris (FR)
Inventeur : Keil, Borivog
25, Chemin des Buttes
F-91190 Gif-sur-Yvette (FR)

(74) Mandataire : Gutmann, Ernest et al
S.C. Ernest Gutmann - Yves Plasseraud 67, boulevard
Haussmann
F-75008 Paris (FR)

**Description**

L'invention est relative à une préparation à activité collagénolytique, plus particulièrement à des compositions pharmaceutiques renfermant cette préparation, en association avec un véhicule pharmaceutique, notamment pour l'utilisation dans le traitement de pathologies se manifestant par une altération incontrôlée des structures riches en collagène, chez l'homme ou l'animal.

Plusieurs types de micro-organismes producteurs d'une enzyme collagénolytique ou « collagénase » ont déjà été décrits dans la littérature. Voir, par exemple le brevet US-A-3 677 900. Il est cependant souvent difficile de contrôler les teneurs de ces préparations en constituants actifs, ces préparations pouvant d'ailleurs n'être pas exemptes d'un certain nombre de constituants toxiques, par exemple des neurotoxines, qui les rendent impropres à l'usage dans ces compositions à destination pharmaceutique.

L'invention a pour but de fournir une composition pharmaceutique permettant une détersion enzymatique sélective à l'égard de structures collagéniques pathogènes, lorsqu'elle est appliquée à des lésions se manifestant par une altération incontrôlée de collagène. Elle a encore pour but la réalisation de compositions dans lesquelles les proportions relatives des principaux constituants de la préparation collagénolytique sont équilibrées selon des proportions prédéterminées en fonction de la qualité du résultat thérapeutique recherché.

La composition pharmaceutique selon l'invention est caractérisée en ce qu'elle contient, en association avec un véhicule pharmaceutique, une dose efficace de principe actif contenant les trois constituants suivants dans des proportions relatives correspondant à :

au moins 0,350 μkat de collagénase, laquelle reconnaît spécifiquement une séquence peptidique X-glycyl-L-prolyl, dans laquelle X est un résidu d'acide aminé naturel lié à l'extrémité N-terminale du résidu glycyle, séquence qu'elle coupe spécifiquement au niveau de la liaison X-glycyl ;

de 20 à 200 unités caséinolytiques pour ce qui est de la teneur en protéases neutres ; et

30 à 500 unités nucléasiques, en ce qui concerne les endonucléases ; et en ce qu'en outre elle est inhibée au moins partiellement par la myosine.

Une collagénase préférée entrant dans la composition pharmaceutique selon l'invention est constituée par celle qui peut être obtenue à partir de cultures de Vibrio alginolyticus chemovar iophagus, dont les caractéristiques taxonomiques principales seront rappelées plus loin, ou de tous micro-organismes dérivés de celui-ci ou capables comme celui-ci de produire une telle collagénase. Plus généralement encore font partie des collagénases susceptibles d'être mises en œuvre dans les compositions pharmaceutiques selon l'invention, celles qui sont capables de réagir avec des anticorps sélectifs préparés contre la collagénase dite « collagénase d'Achromobacter » répertoriée sous le n° EC.3.4.24.8 dans « Enzyme Nomenclature » Edition IUPAC-IUB (1978). Cette collagénase est obtenue à partir de la souche de Vibrio alginolyticus chemovar iophagus qui a été déposée à la Collection Nationale de Culture de Micro-organismes de l'INSTITUT PASTEUR (C.N.C.M.) sous le n° I-029.

Cette collagénase se distingue d'autres collagénases connues par sa haute activité spécifique. Celle-ci peut atteindre 2 unités microkatal/mg (μkat/mg) de protéine. Elle a été décrite, par exemple, dans l'article intitulé « Subunit structure of Achromobacter Collagenase », de V. KEIL-DLOUHA et B. KEIL, paru dans Biochim. Biophys. Acta 522, 218-228 (1978).

On pourra encore se référer, en ce qui concerne la caractérisation chimique de cette enzyme ainsi que du micro-organisme producteur, aux articles intitulés « Chemical characterization and study of the autodigestion of pure collagenase from Achromobacter iophagus » de Vera KEIL-DLOUHA, paru dans Biochimica et Biophysica Acta, 429 (1976) 239-251, et « Some newly characterized collagenases from procaryotes and lower eucaryotes » de Borivoj KEIL, paru dans Molecular & Cellular Biochemistry, January 26 (1979), vol. 23, nr. 2.

De préférence, les protéases et les endonucléases associés à la collagénase sont issues des mêmes micro-organismes, et sont obtenues simultanément avec la collagénase dans les milieux de culture du micro-organisme.

L'invention concerne plus particulièrement des compositions pharmaceutiques contenant des doses exprimées par l'activité de la collagénase, rapportées au poids total de la composition, au moins égales :

à 0,05 μkat/g de la composition pharmaceutique pour les émulsions, pommades, poudres ou toutes compositions non vraiment liquides, au sens habituellement donné à cette expression, et

à 0,05 μkat/ml de la composition pharmaceutique pour les solutions constituées en vue de l'usage topique.

Des pommades préférées selon l'invention contiennent notamment de 0,1 à 2, de préférence de 0,5 à 1 μkat de collagénase par gramme de pommade.

Des solutions pour applications topiques préférées conformes à l'invention contiennent de 0,1 à 2, de préférence 0,5 à 1 μkat de collagénase par ml de solution.

Dans des compositions davantage préférées encore, les proportions relatives les unes vis-à-vis des autres des susdits constituants correspondent respectivement à des activités :

d'au moins 0,350 μkat en ce qui concerne la collagénase,

de 20 à 50 unités caséinolytiques en ce qui concerne les protéases neutres et

de 30 à 60 unités nucléasiques, en ce qui concerne les endonucléases.

L'utilisation des compositions selon l'invention dans le traitement de lésions du genre sus-indiqué (par exemple des brûlures, ulcères, escarres dures ou blanches à base de collagène, chéloïdes, telles que celles produites après des interventions chirurgicales) se révèle particulièrement bénéfique.

Dans son action thérapeutique, la composition selon l'invention décompose préférentiellement le collagène du tissu conjonctif. Elle ne s'attaque pas à la masse musculaire, en particulier aux myofibrilles des muscles, son composé collagénolytique étant inhibé par la myosine du muscle. La composition selon l'invention sera quelquefois, ci-après, dénommée « Achromase ».

Simultanément, l'action de l'Achromase est caractérisée par la production, à partir de substrats macromoléculaires de la nécrose, de fragments qui ont une action chémotactique sur les éléments du sang ; c'est par cette action chémotactique qu'en particulier les macrophages et les leucocytes s'accumulent à la périphérie de la zone d'action de l'Achromase. Cette accumulation des éléments du sang est favorable au processus de régénération tissulaire (par exemple, dans les ulcères, dans les tissus endommagés par des brûlures).

Une autre caractéristique de l'action de l'Achromase à l'égard de tissus altérés à caractère nécrotique est qu'elle s'arrête au niveau des tissus vivants. L'action de l'Achromase s'arrête après avoir décomposé les tissus morts à la limite des tissus vivants. Elle ne provoque pas, par l'action de ses composés protéolytiques, de lésions du tissu vivant. Ces effets se produisent particulièrement grâce aux systèmes d'inhibition qui existent dans les tissus vivants, notamment aux trois systèmes mettant en jeu :

1) des inhibiteurs de la collagénase de nature polypeptidique, présents dans le tissu non endommagé,

2) la myosine présente dans les tissus musculaires, et

3) des inhibiteurs non spécifiques de la collagénase et des protéases, en particulier de la $\alpha_2$-macroglobuline, présents dans la circulation sanguine.

Il est à cet égard important de souligner que pour être efficaces les compositions pharmaceutiques doivent avoir une activité collagénolytique élevée, présentant notamment les ordres de grandeurs qui ont été indiqués plus haut. Il s'est en fait avéré que, contrairement à ce que l'on pouvait craindre, la sélectivité de l'action de la préparation collagénolytique de l'invention à l'égard des seuls tissus nécrosés ou, d'une façon plus générale, de tout tissu non sain, n'était pas affectée même à des doses élevées de collagénase. En effet les barrières naturelles que présentent les tissus sains contre l'action de la collagénase ou les régulateurs de l'activité de celle-ci qu'ils contiennent se révèlent parfaitement efficaces contre ce qui pouvait a priori être considéré comme un « surdosage » de l'enzyme.

Les protéases neutres, qui forment le second constituant actif de l'Achromase, complètent l'action de la collagénase en dégradant davantage encore les fragments du substrat moléculaire collagénolytique initial.

Lorsque la préparation mise en œuvre dans la composition pharmaceutique selon l'invention est originaire de Vibrio alginolyticus chemovar iophagus, lesdites protéases sont dépourvues de constituants indésirables.

Enfin, le troisième constituant de l'Achromase, essentiellement formé d'endonucléases, contribue à la destruction de l'ADN susceptible d'être libéré par les cellules à l'occasion du processus infectieux, donc du pus dont l'ADN libéré forme une partie importante.

Les conditions dans lesquelles les proportions mutuelles de ces constituants peuvent être réglées sont exposées plus loin.

L'invention concerne encore des compositions collagénolytiques préférées dans lesquelles la susdite collagénase est associée avec les préparations efficaces d'hydrolysat de collagène, notamment d'hydrolysat enzymatique du collagène.

Essentiellement ces hydrolysats sont constitués de fragments de collagène, par exemple issus de peaux de veaux constitués par des molécules peptidiques dont les poids moléculaires moyens sont inférieurs à 10 000 daltons, notamment compris entre 6 000 et 8 000 daltons. Ces hydrolysats peuvent encore être caractérisés par certains de leurs amino-acides constitutifs plus particulièrement par la présence d'hydroxy-proline. Par exemple, un hydrolysat de collagène caractéristique comporte les acides aminés suivants (originaire du collagène) dans les proportions suivantes :

— glycine : 33 %
— proline : 8,4 %
— hydroxyproline : 11,4 %

Ces hydrolysats de collagène peuvent être obtenus par tout procédé de fragmentation approprié du collagène dénaturé ou non. Le terme « hydrolysat » n'est pas limité à des produits obtenus par hydrolyse chimique ou enzymatique du collagène dénaturé ou non. Les hydrolysats du genre en question sont par exemple obtenus par atomisation de gélatine.

Il est à remarquer que ces hydrolysats de collagène sont de même nature que les produits résultant de la dégradation du collagène fibreux ou nécrotique contenu dans les plaies et que les compositions collagénolytiques selon l'invention visent à hydrolyser.

Il a été constaté que l'addition à la collagénase de l'invention de proportions substantielles d'hydrolysat de collagène tel que mentionné plus haut se manifeste par un accroissement de l'activité curative des compositions selon l'invention.

Un autre avantage très important de l'addition de ces hydrolysats de collagène aux compositions de collagénase selon l'invention réside dans l'accroissement important de la stabilité au stockage des compositions collagénolytiques selon l'invention. Cet accroissement de stabilité se manifeste avec une ampleur particulière à l'occasion de la lyophilisation de préparations de collagénase conforme à l'invention. Il sera donc particulièrement intéressant d'associer ces hydrolysats de collagène, très solubles à froid, aux préparations de collagénase dans la phase terminale de la préparation de ces dernières. En particulier ces hydrolysats de collagène pourront être ajoutés directement aux solutions de collagénase obtenues à partir des milieux de culture du micro-organisme producteur, notamment après les ultra-filtrations et les opérations de purification supplémentaires requises des solutions obtenues, pour éliminer les constituants insolubles et solubles indésirables contenus dans les milieux de culture éliminés, mais avant la lyophilisation finale des préparations de collagénase obtenues.

Des compositions pharmaceutiques préférées de l'invention contiennent les hydrolysats de collagène et la collagénase à raison de 1 à 25 mg, de préférence 2 à 10 mg d'hydrolysat de collagène pour 1 mg de collagénase. On peut encore exprimer des rapports préférés de la façon suivante. Les compositions préférées contiennent de 1 à 25, notamment de 2 à 10, par exemple 5 mg d'hydrolysat de collagène par microkatal de collagénase.

Il va sans dire que ces intervalles de proportions s'appliquent indistinctement au cas où le collagène est associé ou au cas où il n'est pas associé aux protéases et endonucléases sus-indiquées.

Une composition pharmaceutique préférée à base de trois constituants — collagénase, protéases et endonucléases — est caractérisée en ce qu'elle contient également un hydrolysat de collagène à raison de 1 à 25 parties en poids, de préférence 2 à 10 parties en poids d'hydrolysat de collagène par partie en poids de collagénase.

La composition pharmaceutique selon l'invention joue donc un rôle essentiel au niveau de la destruction des déchets tissulaires qui tendent à s'accumuler dans la région des lésions du genre en question, cette destruction (détersion) devant nécessairement précéder le début de la cicatrisation naturelle ou induite, par exemple par greffe.

L'invention concerne également une composition lyophilisée et stabilisée à base d'une collagénase caractérisée en ce qu'elle contient, en association avec ladite collagénase, de 1 à 25 parties en poids, de préférence 2 à 10 parties en poids d'hydrolysat de collagène par partie en poids de collagénase.

L'invention concerne plus particulièrement une composition lyophilisée et stabilisée caractérisée en ce qu'elle contient une collagénase reconnaissant spécifiquement une séquence peptidique X-glycyl-L-prolyl, dans laquelle X est un résidu d'acide aminé naturel lié à l'extrémité N-terminale du résidu glycyle, séquence qu'elle coupe spécifiquement au niveau de la liaison X-glycyl, et en association avec ladite collagénase, de 1 à 25 parties en poids, de préférence 2 à 10 parties en poids d'hydrolysat de collagène par partie en poids de collagénase.

Une composition lyophilisée et stabilisée préférée à base des trois constituants — collagénase, protéases et endonucléases — est caractérisée en ce qu'elle contient, en association avec les trois constituants, 2 à 10 parties en poids d'hydrolysat de collagène par partie en poids de collagénase.

L'invention concerne encore un procédé de production d'une préparation active conforme à l'invention, contenant plus particulièrement les trois types de constituants qui ont été envisagés ci-dessus. Ce procédé met plus particulièrement en œuvre la bactérie aérobie Vibrio alginolyticus chemovar iophagus. Ce procédé consiste à cultiver ce micro-organisme dans des conditions en soi connues et à induire également de façon en soi connue la production de collagénase par l'addition au milieu de culture d'un inducteur approprié, plus particulièrement de collagène ou de fragments de collagène ayant un poids moléculaire encore suffisant pour former une structure secondaire caractéristique des chaînes hélicoïdales de collagène, à poursuivre cette culture jusqu'après induction dans le milieu de culture d'une production desdites protéases neutres, pouvant être interrompue quand les proportions relatives de collagénase et de protéinase on atteint des valeurs désirées comprises dans les intervalles respectifs sus-indiqués, la préparation active pouvant ensuite être récupérée par des techniques en soi connues à partir du milieu de culture. En particulier, on peut, après élimination des cellules, précipiter les protéines hors du surnageant avec une concentration suffisante de sulfate d'ammonium pouvant par exemple atteindre 60 % du taux de saturation en ce sel de la solution. Le précipité remis en suspension peut être dialysé contre de l'eau distillée pour éliminer les ions entraînés au cours de la précipitation, avant d'être finalement lyophilisé. En variante, le principe actif peut être purifié par ultrafiltration.

Dans ce qui précède, c'est en jouant sur la durée de la culture que l'on a proposé de régler les proportions relatives de collagénase ou de protéase (ou protéinase). Il peut également être envisagé de compléter la collagénase avec des protéinases et endonucléases d'origine extérieure, ou au contraire de retirer l'excédent de protéinase et d'endonucléase pour le cas où cette solution pourrait être purifiée, eu égard au rendement accru en collagénase que l'on pourrait attendre d'une prolongation de la durée de la culture.

Cette dernière opération pourra être mise en œuvre en mettant à profit les masses moléculaires très différentes de la collagénase et des protéinases : en particulier ces masses moléculaires sont supérieures à 30 000 pour la collagénase et inférieures à 30 000 pour les protéases, de sorte que leur séparation est aisée, par exemple sur des membranes ultra-filtrantes appropriées. En particulier, le milieu de culture obtenu après l'interruption de la culture et la séparation des bactéries, peut faire l'objet de trois ultra-filtrations successives : la première en vue d'éliminer les bactéries résiduelles et pour obtenir un liquide clair contenant la collagénase et les protéases, la seconde sur une membrane ultra-filtrante séparant les protéines des poids moléculaires supérieurs, à 30 000, particulièrement les collagénases et la troisième sur une membrane filtrante permettant de séparer les poids moléculaires supérieurs à 10 000, ce qui permet de récupérer les protéases. La collagénase et les protéases peuvent alors être remélangées aux proportions voulues.

Conformément à la caractéristique supplémentaire déjà mentionnée plus haut de l'invention il est avantageux, avant la récupération de la collagénase et, le cas échéant, des protéases et des endonucléases, éventuellement davantage purifiées, notamment par lyophilisation de leurs solutions, d'ajouter au préalable à celles-ci des hydrolysats de collagène, de préférence dans les proportions qui ont été indiquées. En d'autres termes, ce sont ces solutions complétées par les hydrolysats de collagène, qui sont ensuite soumises à lyophilisation.

L'addition de ces hydrolysats de collagène à une collagénase en solution avant sa récupération à partir de cette solution, peut donc également s'analyser comme représentant l'étape essentielle d'un procédé visant à stabiliser ladite collagénase. Ce procédé de stabilisation est donc caractérisé par l'étape que constitue la lyophilisation de la solution contenant la collagénase, en présence des susdits hydrolysats de collagène, pris de préférence dans les proportions relatives qui ont été indiquées plus haut vis-à-vis de la collagénase.

Pour doser les différents constituants de la composition, on peut avoir recours aux techniques décrites ci-après qui mettent en œuvre les substrats, les réactifs et les méthodes de calcul également mentionnés ci-après.

I. Dosage de la collagénase (Wünsch E. et Heidrich H. G. (1963) Z. für Physiol. Chem. 333, 149-151)

Substrat

Le substrat utilisé est du 4-phénylazo-benzyloxy-carbonyl-L-Pro-Leu-Gly-L-Pro-D-Arg. HCl (PZ-PLGPR), commercialisé par la Société Fluka.

Réactifs

Les réactifs mis en œuvre sont les suivants :

A. Tampon

le tampon est constitué à partir des trois solutions suivantes :
— solution A1 : effectuée à l'aide de 160 mM de véronal (sel de sodium de l'acide 5,5-diéthylbarbiturique) et 143 mM d'acétate de sodium, 3 $H_2O$ ;
— solution A2 : HCl 1N ;
— solution A3 : NaCl à 8,5 % ;
le tampon est obtenu en mélangeant 200 ml de solution A1 avec 80 ml de solution A3, et en ajustant le pH à 8,5 avec la solution A2 ; on complète à 1 l en ajoutant de l'eau et on additionne $CaCl_2$, 2 $H_2O$ pour obtenir une concentration finale de 4 mM.

B. Solution de substrat

elle est obtenue en dissolvant 10 mg de PZ-PLGPR dans 100 µl de méthanol ; on ajoute du tampon pour compléter à 10 ml et la solution de substrat ainsi obtenue reste stable une semaine.

C. Echantillon de collagénase

l'échantillon de collagénase est obtenu en conservant 1 mg de solution dans 1 ml de tampon ; à l'aide d'échantillons d'activité spécifique inconnue, on dilue selon les lectures préliminaires ; les dilutions optimales doivent donner les valeurs colorimétriques de la densité optique à 320 nm (voir ci-dessous) dans la gamme de 0,1 à 1,0.

D. Acide citrique

il s'agit d'une solution à 0,5 % dans l'eau.

E. Acétate d'éthyle

Technique de dosage

Elle se déroule comme suit :

1. on préchauffe la solution B à 37 °C ;

2. on mélange 0,1 ml de solution de collagénase (correctement diluée) avec 0,4 ml de solution B préchauffée et on laisse incuber pendant 15 mn à 37 °C ;

3. on additionne 1 ml de solution D et on mélange ;

4. on additionne 5 ml d'acétate d'éthyle et on mélange vigoureusement ;

5. on enlève ensuite la couche organique supérieure et on sèche en additionnant $Na_2SO_4$ anhydre ;

6. on mesure la densité optique à 320 nm par rapport à celle d'un témoin obtenu dans les mêmes conditions que l'échantillon étudié, selon la procédure indiquée sous les points 1 à 5, si ce n'est qu'on utilise du tampon à la place du substrat (solution B).

Méthode de calcul

$$1 \text{ unité Wünsch-Heidrich} = \frac{\text{Densité optique à 320 nm (relative à 0,1 ml d'échantillon)}}{0,044}$$

$$\text{Activité spécifique de l'échantillon exprimée en katals} = \frac{0,2525 \times \text{densité optique à 320 nm en nkat/mg}}{mg}$$

1,0 μkat = 90 000 unités de WH

Le coefficient est déduit de la courbe d'étalonnates obtenue avec le produit qui découpe le peptide (PZ-Pro-Leu) (composé B commercialisé par Fluka).

II. Dosage des protéases (Laskowski M. (1955) Methods in enzymology II, 32 ; Kunitz M. (1947) J. Gen. Physiol. 30, 291).

Substrat

Le substrat utilisé est de la caséine pour usage biochimique (commercialisé par Merck, sous le n° 2 244).

Réactifs

Les réactifs utilisés sont les suivants :
— solution A :
elle est constituée par :
Aa : acide borique 0,2 M (12,4 g/1 000 ml) ;
Ab : borax 0,05 M (19,05 g/1 000 ml) ;
pour obtenir la solution A, on ajuste 100 ml de solution Aa à pH 7,6 à l'aide de solution Ab (environ 4 ml) ;
— solution B :
on met en suspension 1 g de caséine dans 100 ml de solution A ; on chauffe dans un bain d'eau à 100 °C jusqu'à dissolution totale (environ 15 mn) ; la solution opalescente ainsi obtenue peut être conservée pendant une semaine à 4 °C ;
— solution C :
acide trichloroacétique à 5 % dans l'eau.

Echantillon de protéinase

La dilution des échantillons contenant la protéinase doit être ajustée selon le dosage préliminaire jusqu'à un équivalent de 2-10 μg de trypsine/ml.

Technique de dosage

Elle se déroule comme suit :

1. on prépare les tubes (tubes de plastique Vidal 11 × 70), en nombre correspondant au double du nombre d'échantillons, afin qu'à chaque échantillon corresponde son propre témoin ;

2. on fait pré-incuber 0,5 ml de solution B dans chaque tube, pendant 5 mn, à 37 °C ;

3. à chaque tube « d'échantillon », on ajoute 0,5 ml d'échantillon ; on fait incuber 20 mn à 37 °C sous agitation ; on additionne 1,5 ml de solution C ;

4. à chaque tube témoin, on additionne 1,5 ml de solution C ; on fait incuber 5 mn à 37 °C et on ajoute 5 ml d'échantillon ;

5. on laisse tous les tubes à 4 °C, toute la nuit ;

6. on centrifuge pendant 10 mn à 8 000 tr/mn à température ambiante ;

7. on lit la densité optique à 280 (DO$_{280}$) du surnageant de l'échantillon vis-à-vis du témoin correspondant.

Calcul

1. Courbe d'étalonnages :

on dilue une solution stockée de trypsine pure (0,1 mg/ml d'HCl 10$^{-3}$ M) pour obtenir des concentrations finales de 1, 2, 4, 6, 8 μg de trypsine par ml ; on effectue ensuite les étapes 1 à 7 mentionnées dans le paragraphe précédent.

2. Une unité d'activité correspond à 1 μg de trypsine ; une unité d'activité spécifique correspond à 1 μg de trypsine par mg de protéine.

III. Dosage de la déoxyribonucléase (Kunitz M. (1950) J. Gen. Physiol. 33, 349-363)

Réactifs

Les réactifs utilisés sont les suivants :
— déoxyribonucléase I (commercialisée par BOEHRINGER) de qualité II (2 000 U/mg) ;
— acide déoxyribonucléique (commercialisé par BOEHRINGER) à 3 ml/10 mg.

Tampon

Le tampon est constitué comme suit : 100 mM de Tris. HCl ; 4 mM de MgSO$_4$ ; 7 H$_2$O ; 1,8 mM de CaCl$_2$ · 2 H$_2$O ; on ajuste à pH 7,5 avec de l'acide chlorhydrique.

Solutions

— Enzyme standard : on dissout 1 mg de déoxyribonucléase I dans 1 ml de tampon.
— Substrat : on dilue 150 μl dans 50 ml de tampon.

Technique de dosage

Elle se déroule comme suit :
1. on ajuste au 0 l'absorbance à 260 nm de 3 ml de solution de substrat ;
2. on ajoute 100 μl de l'enzyme standard et on mélange ;
3. on enregistre la variation d'absorbance pendant 10 mn ;
4. on détermine le rapport $\Delta A_{260}$ (variation d'absorbance à 260 nm)/mn ;
5. on recommence les étapes 1 à 4 avec une solution de substrat frais et un échantillon d'enzyme inconnu et on détermine la valeur $\Delta A_{260}$/mn.

Calcul

mg/ml = densité optique à 280 mn (DO$_{280}$) × 0,9

Unités par mg

$$\text{de protéine} = \frac{\Delta A/\text{mn} \times 1\,000 \times 6}{\text{mg d'enzyme utilisée}}$$

IV. Dosage des protéines (Lowry H. O., Roseborough N. J. et Randall R. J. (1951), J. Biol. Chem. 193, 265)

Réactifs

Les réactifs utilisés sont les suivants :
— solution A : Na$_2$CO$_3$ à 2 % dans NaOH 0,1 M ;
— solution B : Na à 2 %, tartrate de potassium ;
— solution C : CuSO$_4$ à 1 % ;
— solution D : 1 ml de solution B + 1 ml de solution C ; on dilue à 100 ml avec la solution A ;
— solution E : réactif de foline dilué à 1/1.

Courbe d'étalonnages

1. On prépare la solution de protéine de référence de 1 mg de sérum albumine bovine (BSA,

7

commercialisée par Sigma) dans 2 ml d'eau ;

2. on prépare une série de dilutions de solution de référence de 10 à 80 µl à un volume final de 400 µl dans l'eau ;

3. on ajoute 2 ml de solution D, on mélange et on laisse 10 mn à la température du laboratoire ;

4. on additionne 200 µl de solution E ; on laisse incuber à l'obscurité à 50 °C pendant 10 mn ;

5. on lit la densité optique à 750 nM vis-à-vis du témoin (eau au lieu de la solution de BSA utilisée dans l'étape 2°) ; on construit ainsi la courbe d'étalonnages.

Dosage

On traite l'échantillon inconnu à différentes dilutions (volume final 400 µl, étape 2°).

Calculs

On exprime le contenu protéique de l'échantillon inconnu comme équivalent en poids de sérum albumine bovine (BSA) de référence.

D'autres caractéristiques de l'invention apparaîtront encore au cours de la description qui suit des conditions de culture de Vibrio alginolyticus chemovar iophagus. Référence sera faite aux figures dans lesquelles :

la fig. 1 contient des courbes représentatives des concentrations relatives de différentes enzymes dont les productions sont induites à l'occasion de la culture de la susdite bactérie en fonction du temps ;

la fig. 2 comprend des courbes comparatives visant à mettre en évidence le caractère inhibiteur de la myosine vis-à-vis de la collagénase.

La souche Vibrio alginolyticus chemovar iophagus (I-029) est aérobie — anaérobie facultatif — et se présente sous forme de bâtonnets gram négatif de 2-2,5 µm de long et de 1-1,5 µm de large, mobiles au moyen de flagelles polaires simples. Elle forme des colonies rondes et jaunes de 2-3 mm de diamètre, après incubation à 30° C pendant une nuit sur un milieu de thiosulfate-citrate-bile-sucrose (TCBS).

Elle présente une réponse positive dans les tests suivants : oxydase de cytochrome, réaction de Voges-Proskauer, production d'indole, décarboxylase de lysine, gélatinase (test de pellicule), estérase de l'agent mouillant commercialisé sous la désignation TWEEN 80, réductase de tétrathionate, réduction de nitrate, production d'acide à partir de glucose, maltose, mannitol, sucrose, tréhalose, mannose et glycérol, utilisation de citrate (Simmons). Elle est sensible à l'action des antibiotiques aminoglycosidiques, au chloramphénicol, aux tétracyclines, à la colistine, à la rifampicine, aux sulfonamides, à l'acide nalixide, à la nitrofurantoïne et à l'agent vibriostatique 0/129 dans le test du disque, mais elle résiste à la triméthoprime.

Elle donne une réponse négative dans les tests suivants : réaction au rouge de méthyle, dihydrolase d'arginine, uréase, phénylalanine déaminase, test ONPG, production d'H$_2$S (agar fer Kligler), production de gaz de glucose, production d'acide à partir de xylose, d'arabinose, d'adonitol, de rhamnose, de sorbose, de sorbitol, de dulcitol, de lactose, d'inositol, de salicine, de raffinose, de mellibiose, d'α-méthylglucoside, de mucate. Elle n'utilise pas le malonate.

La souche I-029 est très halophile avec une croissance optimale qui se produit dans du chlorure de sodium à 13 % et une tolérance apparente au chlorure de sodium à 15 %. Elle est également pleinement résistante à l'ampicilline, à la carbénicilline, en partie à la céphalotine. Elle présente des réactions positives dans les tests à la décarboxylase ornithine et dans les tests de fermentation de cellobiose.

Conditions de culture

Les cellules sont cultivées sous agitation et aération forcée (1,4 atmosphères i. e. 141,855 KPa) à 30 °C dans un milieu contenant Tris. HCl 0,1 M, NaCl 0,4 M, 2 mM de CaCl$_2$, à pH 7, contenant soit des acides casaminés à 2,5 % (Difco Labs., Detroit, Mich.). Deux heures après le début de la culture, on ajoute au milieu la composition commercialisée sous la désignation ASF (fragments de peptide de collagène de peau de veau ; poids moléculaire moyen 7000, Rousselot S.A., France) pour obtenir une concentration de 1,5 % en poids. Pour les contrôles de croissance, la turbidité est mesurée par l'absorbance par cm$^{-1}$ à 600 nm dans des échantillons extraits à une heure d'intervalle.

Production de collagénase, de protéinase et de nucléase

La souche produit de la protéinase caséinolytique de la nucléase extra-cellulaire et, dès que l'ASF a été ajouté, de la collagénase. Le niveau d'activité de la collagénase s'élève alors jusqu'à atteindre 60 nkat/ml.

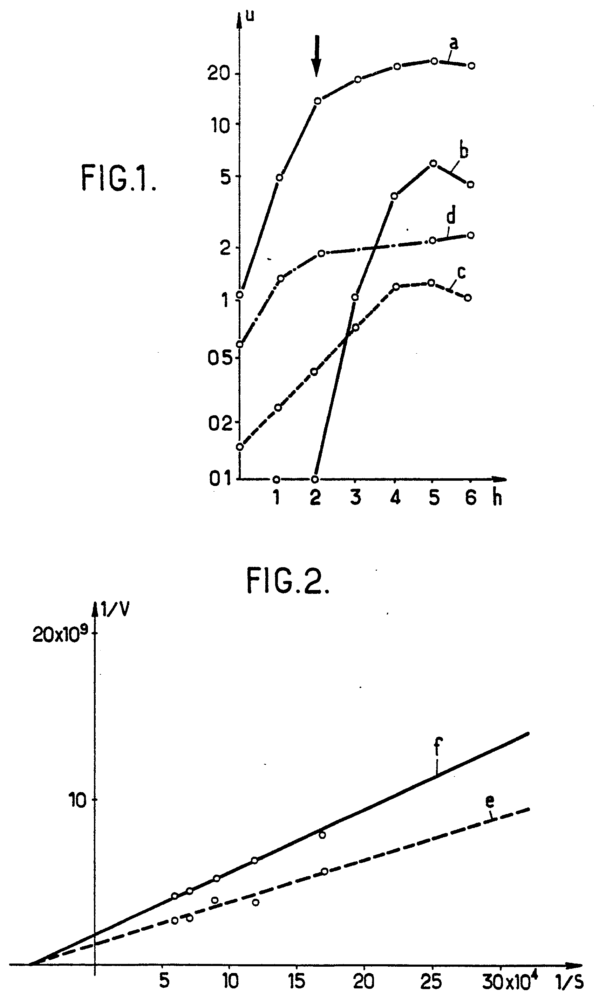

Les courbes de la fig. 1 sont représentatives en ce qui concerne :

la courbe a, de la croissance cellulaire,

la courbe b, de l'activité de la collagénase,

la courbe c, de l'activité de la protéinase caséinolytique et

la courbe d, de l'activité de la nucléase, en fonction du temps.

La culture est de préférence interrompue, lorsque l'activité de la collagénase passe par un maximum, notamment au bout de 5 heures dans le cas représenté. La collagénase est concentrée et récupérée à partir de ce milieu. Elle peut être davantage purifiée comme décrit dans les ouvrages de KEIL-DLOUHA, V. 1976, « Chemical characterization and study of the autodigestion of pure collagenase from Achromobacter iophagus, Biochem. Biophys. Acta, 429, 239-305 et de LECROISEY A., V. KEIL-DLOUHA, D.R. WOODS, D. PERRIN et B. KEIL, 1975, « Purification, stability and inhibition of the collagenase from Achromobacter iophagus, FEBS Letters 59, 167-172.

Une préparation type d'Achromase contient 0,312 mg de protéine (poids sec) par mg du produit total. Elle se caractérise par les activités relatives suivantes, par mg de poids sec de protéine :
— collagénase : 0,554 μkat
— protéases : 145 unités caséinolytiques
— endonucléases : 477 unités nucléasiques

Stabilisation de l'activité collagénolytique

L'introduction dans une solution de collagénase d'hydrolysat de collagène a pour effet de stabiliser l'activité collagénolytique lorsque la préparation est lyophilisée. Les effets de cette stabilisation, exprimés en pourcentages de préservation de l'activité enzymatique d'une préparation de collagénase, avant et après lyophilisation, ont été appréciés dans des essais dans lesquels on a mis en œuvre des proportions variables d'hydrolysat de collagène vis-à-vis d'une dose déterminée de collagénase. L'hydrolysat de collagène utilisé étant celui commercialisé en France sous la désignation ASF par les Etablissements Rousselot S.A. Il s'agissait d'une gélatine atomisée soluble à froid dans l'eau.

Les résultats obtenus sont indiqués dans le tableau ci-après pour les proportions relatives d'Achromase et d'ASF qui résultent de la colonne de gauche du tableau.

Activité enzymatique en %

| Achromase : ASF | Avant lyophilisation | Après lyophilisation |
|---|---|---|
| 1 : 0 | 100 | 50 |
| 1 : 1 | 100 | 63 |
| 1 : 5 | 100 | 96,6 |
| 1 :10 | 100 | 94 |
| 1 :25 | 100 | 78 |

Il résulte de l'examen de ce tableau que :
— la lyophilisation d'une solution d'Achromase, en l'absence d'ASF entraîne une perte de 50 % de l'activité collagénolytique et
— l'addition d'ASF réduit sensiblement les pertes d'activité.

Des résultats particulièrement favorables sont observés pour des proportions de 2 à 10 parties en poids d'ASF par rapport à une partie du poids d'Achromase. Pour la proportion de 5 parties d'ASF pour une partie d'Achromase on constate une préservation quasi totale de l'activité enzymatique de la solution initiale.

A la préservation de l'activité enzymatique au cours de la lyophilisation, s'ajoute encore la remarquable stabilité qui peut être conférée par les hydrolysats de collagène aux préparations de collagénase contenant de 1 à 10 partie en poids d'hydrolysat de collagénase lyophilisée, à l'occasion de leur stockage. Il a ainsi été constaté que la perte d'activité collagénolytique, en %, à la température de 4 °C, était de 25 % pour une collagénase exempte d'hydrolysat de collagène au bout de 7 mois. Au terme du même délai et à la même température l'activité collagénolytique de préparations de collagénase contenant de 1 à 10 parties en poids d'hydrolysat de collagène pour une partie en poids de collagénase était conservée à 100 %.

Les mêmes effets de stabilité réalisés à la température de 20 °C au lieu de 4 °C ont conduit au bout de 7 mois à une perte d'activité de 30 % pour la préparation de collagénase exempte d'hydrolysats collagénolytiques et de 5 % seulement pour des préparations contenant les mêmes proportions d'hydrolysat collagénolytique que ci-dessus.

Des stabilisations semblables ont été observées sur d'autres types de préparations à base de

9

# 0 115 974

collagénase (ou d'Achromase), notamment lorsque celles-ci se présentent sous des formes prêtes à l'emploi, telles que celles dont il sera question plus loin.

Propriétés biologiques

Les préparations de collagénase obtenues présentent, outre leur activité collagénolytique sus-indiquée, la caractéristique d'être inhibées par la myosine, laquelle joue le rôle d'un inhibiteur non compétitif pour la collagénase. Cette propriété peut être mise en œuvre en ayant recours à la technique mettant en jeu l'étude cinétique de l'effet de la myosine sur l'activité de la collagénase vis-à-vis du collagène.

La dégradation du collagène par la collagénase est suivie par le dosage de l'hydroxyproline libérée après hydrolyse des fragments de collagène solubilisés par la digestion.

L'hydroxyproline étant un acide aminé spécifique du collagène, les autres protéines contenues dans le mélange réactionnel n'interfèrent pas lors du dosage.

Le mélange ci-dessous est incubé au sein d'un tampon Tris, HCl, 0,02 M, NaCl 0,23 M, $CaCl_2$ $5 \cdot 10^{-3}$ M, pH 7,4 pendant une heure à 37 °C :
- collagène musculaire en fibre de 5 à 30 mg,
- collagénase à 0,1 mg/ml dans du $CaCl_2$ $10^{-4}$ : 1 ml ;
- tampon d'incubation ou
- suspension de myosine à 5 mg/ml dans ce même tampon : 5 ml.

Après incubation, le mélange réactionnel est centrifugé 5 minutes à 12 000 g 0,5 ml de surnageant sont hydrolysés une nuit à 110 °C avec 0,5 ml d'HCl 12 N. Les échantillons sont ensuite évaporés sous vide et repris par un volume d'eau tel que le dosage de l'hydroxyproline soit rendu possible.

Le résultat d'un tel essai avec une collagénase titrant 0,098 µkat/ml est illustré par les courbes de la fig. 2, dans laquelle la courbe e est représentative des variations du rapport 1/v (v représentant le nombre de moles de collagène digérés par heure à 37 °C) en fonction du rapport 1/s (s représentant le nombre de moles de collagène mis en œuvre), mis en présence de la collagénase, en l'absence de myosine (interprétation selon LINWEAVER BURK).

La courbe f est représentative de l'évolution de la digestion des mêmes préparations respectivement en présence de 5 mg de myosine.

L'examen de ces courbes fait apparaître les effets très significatifs de l'inhibition de l'activité des préparations de collagénase considérés vis-à-vis du collagène.

La collagénase mise en œuvre selon l'invention est dépourvue de toute toxicité. Ceci peut être montré par l'étude des doses léthales effectuée sur la souris selon la méthode de J. T. LICHTFIELD et F. W. WILCOXON.

Les DL-50 mesurées avec une préparation injectée par voie intraveineuse à activité collagénolytique selon l'invention, titrant 0,17 µkat/mg de protéine, ont été les suivantes :
8,58 mg par kg de souris, ce qui correspond à
1,46 µkat par kg de souris, lorsque l'on rapporte cette dose léthale aux unités d'activité collagénolytique de la préparation.

La remarquable activité collagénolytique des préparations selon l'invention a été démontrée dans les tests pharmacologiques in vivo indiqués ci-après.

Ces tests ont consisté dans le traitement avec une préparation contenant la collagénase selon l'invention de brûlures préalablement effectuées sur la peau de porcs, par application d'eau à la température de 80 °C, en plusieurs points du dos de ces animaux pendant 15 secondes (6 lésions sur le côté droit et 6 lésions sur le côté gauche des porcs). L'épiderme mort est alors enlevé des surfaces ébouillantées et les lésions ont été exposées à l'air pendant 48 heures. Chaque lésion avait un diamètre de l'ordre de 3,8 cm. Les essais effectués avec la pommade contenant la collagénase ont été doublés d'essais effectués avec une pommade analogue, mais qui était dépourvue de collagénase, cette pommade ayant été utilisée à titre de témoin.

Les compositions de ces pommades étaient les suivantes :
Pour la pommade témoin :

| | |
|---|---|
| — alcool polyoxyéthylénique | 13 g |
| — huile de vaseline | 20 g |
| — eau | 100 g |
| — hydroxyle de sodium pour régler le pH à 7. | |

La pommade contenant la collagénase contient les mêmes excipients, et 0,25 µkat de collagénase par g de pommade.

La pommade contenant la collagénase a été appliquée sur les lésions du côté droit des porcs, la pommade témoin sur celles du côté gauche.

Les applications de pommade ont été répétées respectivement 3 jours, 4 jours, 6 jours et 13 jours après la réalisation des brûlures.

On a fait les observations suivantes 6 jours et 13 jours après la réalisation des brûlures :

10

Etat des brûlures 6 jours plus tard

La profondeur de la brûlure et l'épaisseur du tissu qui a survécu sont approximativement les mêmes dans les brûlures traitées par la pommade et celles ayant reçu la composition témoin.

Dans les brûlures traitées avec la pommade témoin, on constate la présence d'escarres contenant des fibres intactes de collagène sur les 6 brûlures. Les brûlures traitées avec la pommade contenant la collagénase selon l'invention n'en comportent aucune.

On constate la présence de cellules inflammatoires infiltrées dans la partie supérieure du derme sur les 6 brûlures traitées par la pommade à la collagénase. La même observation ne peut être faite que sur 3 brûlures témoins. L'afflux de cellules inflammatoires témoigne d'un processus de cicatrisation plus avancé que dans les brûlures où ces infiltrations n'ont pas eu lieu.

On ne constate cependant que peu de différences au niveau de la réparation de l'épiderme et du tissu conjonctif, entre les brûlures traitées et les brûlures témoins, sauf que cette réparation paraît un peu plus avancée dans le cas des brûlures traitées.

Etat des brûlures 13 jours plus tard

On constate chez les animaux traités que les brûlures sont recouvertes d'une mince pellicule de tissu dégradé. Du tissu conjonctif nouveau s'est développé sur la totalité de la surface des brûlures, immédiatement sous cette pellicule ainsi que plus en profondeur, dans le derme. L'épiderme s'est développé sous la forme d'un tissu de granulation à partir des bords des lésions, immédiatement sous la pellicule de tissu dégradé.

On constate dans les lésions traitées que la partie collagène des escarres a été dissoute par la collagénase, de sorte que la régénération du tissu conjonctif a pu se développer en surface, dans des conditions analogues à celles qui sont observées dans le cas de blessures par coupures. L'épiderme formé peut migrer sur la surface de ce tissu de granulation.

On constate au contraire que les brûlures témoins sont recouvertes d'escarres profondes de collagène compact qui adhère au tissu et le recouvre. Il s'est formé du tissu conjonctif nouveau seulement sous la nouvelle couche d'épiderme qui s'est formée sur les seuls bords des brûlures. L'épiderme a migré à partir des bords de la lésion, à travers le derme, sous les escarres susdites, séparant ainsi celles-ci du tissu profond. Donc les sources d'épidermes nouveaux ne se constatent qu'au bord des lésions, ce qui requérait une greffe chirurgicale d'épiderme pour assurer la cicatrisation complète de ces lésions.

L'un des avantages de la pommade contenant la collagénase réside dans le fait que le traitement enzymatique réalise automatiquement un équilibre correct entre les tissus nécrotiques et les tissus viables, équilibre qu'il serait difficile de recréer par la voie chirurgicale.

Les préparations pharmaceutiques selon l'invention peuvent se présenter sous toutes les formes susceptibles d'être mises en œuvre par application externe : gels, émulsions, notamment telles que pommades et crèmes, ou solutions ou formes pulvérisables (aérosols en poudre), la collagénase étant dans chaque préparation associée à des excipients conformes selon le cas à chacune desdites formes. Dans le cas de solutions pour l'application topique, on aura recours avantageusement à une mise en solution de la collagénase dans un milieu isotonique, de préférence un tampon physiologiquement acceptable de pH 7-8,5, et de préférence stérile.

Les préparations de collagénase sous forme de solutions au sein d'un véhicule stérile injectable peuvent être administrées par la voie sous-cutanée, à proximité des plaies à traiter ou des chéloïdes. On peut également envisager l'utilisation de ces solutions injectables, pour les administrations profondes, notamment en vue de la destruction de l'accumulation pathologique du collagène dans les tissus, comme par exemple dans les disques intervertébraux.

Les compositions pharmaceutiques selon l'invention sont donc applicables à tous types de lésions se manifestant par une altération incontrôlée des structures riches en collagène. A titre d'exemple de lésions qui peuvent être traitées, on citera les brûlures, ulcères, escarres, les escarres dures ou blanches à base de collagène, chéloïdes, telles que celles produites après des interventions chirurgicales, nécroses, en particulier dans le cas des décubitus ou des ulcères. Les compositions pharmaceutiques selon l'invention peuvent également être appliquées à titre préventif, notamment préalablement à des interventions chirurgicales visant à réaliser des greffes plastiques ou autres, ou à des interventions de chirurgie esthétique, et d'une façon générale à toutes blessures de la peau.

Les solutions de collagénase selon l'invention peuvent aussi être appliquées au traitement des caries dentaires. On sait en effet que la pulpe dentaire est essentiellement constituée de collagène calcifié compact, la carie dentaire se manifestant par une fissuration ou un perçage de la dent, par lequel s'échappe le calcium. La trame décalcifiée de collagène qui subsiste devient alors poreuse et risque de devenir le siège d'une infection bactérienne.

Par introduction d'une solution concentrée de collagénase conforme à l'invention dans cette trame, on induit la dissolution du collagène poreux qui peut alors être éliminé par rinçage. L'action de la collagénase s'interrompt d'elle-même au niveau du collagène calcifié sain.

La solution de collagénase utilisée, tamponnée à pH 7-8,5, notamment au borate, contient de

0 115 974

préférence au moins 1 μkat de collagénase par ml de solution. A cette concentration et a fortiori pour des concentrations plus élevées, le collagène poreux est rapidement dissous.

Après rinçage et désinfection, la dent traitée peut être refermée en mettant en œuvre des techniques bien connues de l'homme de l'art. Les traces résiduelles possibles de collagénase retenues dans la dent sont progressivement inhibées par le pH acide dominant qui se réinstalle au cœur de la dent.

Comme il va de soi et comme il résulte d'ailleurs déjà de ce qui précède, l'invention ne se limite nullement aux cas qui ont été envisagés dans ce qui précède. Elle en embrasse au contraire toutes les variantes, notamment celles où les propriétés stabilisantes des hydrolysats de collagène sont mises en œuvre à l'égard de toutes autres collagénases susceptibles d'être mises en œuvre dans la constitution de compositions pharmaceutiques, dès lors que ces collagénases présentent à la fois les activités sélectives qui ont été définies dans ce qui précède et l'innocuité désirable aux doses suffisantes auxquelles l'activité collagénolytique sélective est susceptible de se manifester. A ce titre l'invention concerne toute association d'une collagénase répondant aux conditions qui précèdent, quelle que soit la source, notamment les cultures de microorganismes à partir desquelles elle a été obtenue, en association avec des hydrolysats de collagène. Ces compositions associant les deux types de principes, notamment dans des proportions équivalentes aux intervalles de proportions préférés qui ont été indiqués, constituent des équivalents des associations plus particulièrement revendiquées dans certaines des revendications qui suivent.

De la même façon les revendications plus particulièrement relatives aux procédés de stabilisation d'une collagénase et plus particulièrement de l'Achromase, notamment par lyophilisation de leurs solutions en présence d'hydrolysats de collagène étendent leurs effets à la stabilisation de toute forme de collagénase, notamment à l'occasion des lyophilisations de leurs solutions, dès lors que seraient mis en œuvre de la même manière des hydrolysats de collagène, en particulier dans les intervalles de proportions relatives définis plus haut.

## Revendications

1. Composition pharmaceutique, notamment pour l'utilisation dans le traitement de pathologies se manifestant par une altération incontrôlée des structures riches en collagène chez l'homme ou l'animal, cette composition étant caractérisée en ce qu'elle contient, en association avec un véhicule pharmaceutique, une dose efficace de principe actif contenant les trois constituants suivants dans des proportions relatives correspondant à :

au moins 0,350 μkat de collagénase, laquelle reconnaît spécifiquement une séquence peptidique X-glycyl-L-prolyl, dans laquelle X est un résidu d'acide aminé naturel lié à l'extrémité N-terminale du résidu glycyle, séquence qu'elle coupe spécifiquement au niveau de la liaison X-glycyl,

de 20 à 200 unités caséinolytiques pour qui est de la teneur en protéases neutres et

30 à 500 unités nucléasiques, en ce qui concerne les endonucléases,

et en ce qu'en outre elle est inhibée au moins partiellement par la myosine.

2. Composition selon la revendication 1, caractérisée en ce que le principe actif est issu de cultures de Vibrio alginolyticus chemovar iophagus.

3. Composition selon la revendication 2, caractérisée en ce que le principe actif est issu de la souche déposée à la C.N.C.M. sous le n° I-029.

4. Composition selon l'une quelconque des revendications 1 à 3, caractérisée en ce qu'elle contient des doses exprimées par l'activité de la collagénase, rapportées au poids total de la composition, au moins égales :

à 0,05 μkat/g de la composition pharmaceutique pour les émulsions, pommades, poudres ou toutes compositions non vraiment liquides, au sens habituellement donné à cette expression et

à 0,05 μkat/ml de la composition pharmaceutique pour les solutions constituées en vue de l'usage topique.

5. Composition selon la revendication 4, caractérisée en ce qu'elle contient de 0,1 à 2, de préférence 0,5 à 1 μkat/g d'émulsions, pommades, poudres ou compositions non vraiment liquides ou de 0,1 à 2, de préférence de 0,5 à 1 μkat de collagénase par ml de solution.

6. Composition selon les revendications 1 à 5, caractérisée par des proportions relatives des susdits constituants correspondant respectivement à des activités :

d'au moins 0,350 μkat en ce qui concerne la collagénase,

de 20 à 50 unités caséinolytiques en ce qui concerne les protéases neutres et

30 à 60 unités nucléasiques, en ce qui concerne les endonucléases.

7. Composition pharmaceutique, notamment pour l'utilisation dans le traitement de pathologies se manifestant par une altération incontrôlée de structures riches en collagène chez l'homme ou l'animal, caractérisée en ce qu'elle contient, en association avec un véhicule pharmaceutique, une dose efficace d'une collagénase reconnaissant spécifiquement une séquence peptidique X-glycyl-L-prolyl, dans laquelle X est un résidu d'acide aminé naturel lié à l'extrémité N-terminale du résidu glycyle, séquence qu'elle coupe spécifiquement au niveau de la liaison X-glycyl, en ce qu'en outre elle est inhibée au moins partiellement par la myosine, caractérisée également par le fait qu'elle contient également un hydrolysat

12

de collagène à raison de 1 à 25 parties en poids, de préférence 2 à 10 parties en poids d'hydrolysat de collagène par partie en poids de collagénase.

8. Composition selon la revendication 7, caractérisée en ce que la collagénase est issue de cultures Vibrio alginolyticus chemovar iophagus.

9. Composition pharmaceutique selon la revendication 8, caractérisée en ce que la collagénase est issue de la souche déposée à la C.N.C.M. sous le n° I-029.

10. Composition pharmaceutique selon les revendications 1 à 6, caractérisée en ce qu'elle contient également un hydrolysat de collagène à raison de 1 à 25 parties en poids, de préférence 2 à 10 parties en poids d'hydrolysat de collagène par partie en poids de collagénase.

11. Composition pharmaceutique selon la revendication 10, caractérisée en ce que la collagénase est issue de cultures de Vibrio alginolyticus chemovar iophagus.

12. Composition pharmaceutique selon la revendication 10, caractérisée en ce que la collagénase est issue de la souche déposée à la C.N.C.M. sous le n° I-029.

13. Composition lyophilisée et stabilisée à base d'une collagénase caractérisée en ce qu'elle contient, en association avec ladite collagénase, de 1 à 25 parties en poids, de préférence 2 à 10 parties en poids d'hydrolysat de collagène par partie en poids de collagénase.

14. Composition selon la revendication 13, caractérisée en ce qu'elle contient une collagénase reconnaissant spécifiquement une séquence peptidique X-glycyl-L-prolyl, dans laquelle X est un résidu d'acide aminé naturel lié à l'extrémité N-terminale du résidu glycyle, séquence qu'elle coupe spécifiquement au niveau de la liaison X-glycyl, et en association avec ladite collagénase, de 1 à 25 parties en poids, de préférence 2 à 10 parties en poids d'hydrolysat de collagène par partie en poids de collagénase.

15. Composition selon la revendication 13, caractérisée en ce que la collagénase qu'elle contient est issue de cultures de Vibrio alginolyticus chemovar iophagus.

16. Composition selon la revendication 13, caractérisée en ce que la collagénase est issue de la souche déposée à la C.N.C.M. sous le n° I-029.

17. Composition lyophilisée et stabilisée à base de trois constituants — collagénase, protéases et endonucléases — selon la revendication 1, caractérisée en ce qu'elle contient, 2 à 10 parties en poids d'hydrolysat de collagène par partie en poids de collagénase.

18. Composition selon la revendication 17, caractérisée en ce que les trois constituants sont issus de cultures de Vibrio alginolyticus chemovar iophagus.

19. Composition selon la revendication 17, caractérisée en ce que les trois constituants sont issus de la souche déposée à la C.N.C.M. sous le n° I-029.

20. Procédé pour l'obtention à partir d'une solution aqueuse de collagénase d'une préparation lyophilisée de collagénase, selon les revendications 13 à 19, sans perte d'activité collagénolytique, caractérisée par le fait que la lyophilisation de ladite solution est réalisée en présence de proportions efficaces d'hydrolysat de collagène préalablement introduit dans la solution.

21. Procédé selon la revendication 20, caractérisé en ce que les proportions d'hydrolysat de collagène préalablement introduit dans la solution vis-à-vis de sa teneur en collagénase sont de 1 à 25 parties en poids, de préférence 2 à 10 parties en poids d'hydrolysat de collagène vis-à-vis d'une partie en poids de collagénase.

22. Composition selon l'une quelconque des revendications 1 à 19, se présentant sous la forme d'une solution pour l'utilisation dans le traitement de caries dentaires ou d'autres altérations de la pulpe dentaire chez l'homme ou l'animal.

**Claims**

1. Pharmaceutical composition, particularly for use in the treatment of pathologies manifested by an uncontrolled alteration of collagen — rich structures in man or animal, this composition being characterized in that it contains in association with a pharmaceutical vehicle, an efficient amount of active principle containing the three following constituents in relative proportions corresponding to :
   at least 0,350 µkat of collagenase, which specifically recognizes a peptidic sequence X-glycyl-L-prolyl, in which X is a natural amino acid residue linked to the N-terminal end of the glycyl residue, said collagenase cutting said sequence specifically at the level of the X-glycyl linkage,
   from 20 to 200 caseinolytic units as regards the neutral proteases content and
   30 to 500 nucleasic units, as regards the endonucleases,
   and by the fact that it is at least particularly inhibited by myosine.

2. Composition according to claim 1, characterized in that the active principle is from cultures of Vibrio alginolyticus chemovar iophagus.

3. Composition according to claim 2, characterized in that the active principle is from the strain deposited at the C.N.C.M. under number I-029.

4. Composition according to anyone of claims 1 to 3, characterized in that it contains amounts expressed by the collagenase activity, with respect to the total weight of the composition, at least equal to :
   0,05 µkat/g of the pharmaceutical composition for the emulsions, pomades, powders or any composition not really liquid, in the usual meaning of this therm and

0,05 μkat/ml of the pharmaceutical composition for the solutions constituted for topical use.

5. Composition according to claim 4, characterized in that it contains from 0,1 to 2, preferably 0,5 to 1 μkat/g of emulsions, pomades, powders or compositions not really liquid or from 0,1 to 2, preferably from 0,5 à 1 μkat of collagenase per ml of solution.

6. Composition according to claims 1 to 5, characterized by relative proportions of the above said constituents corresponding respectively to activities :

of at least 0,350 μkat as regards collagenase,

from 20 to 50 caseinolytic units as regards neutral proteases and

30 to 60 nucleasic units, as regards endonucleases.

7. Pharmaceutical composition, particularly for use in the treatment of pathologies manifested by an uncontrolled alteration of collagen — rich structures in man or animal, this composition being characterized in that it contains in association with a pharmaceutical vehicle, an efficient amount of collagenase specifically recognizing a peptidic sequence X-glycyl-L-prolyl, in which X is a natural amino acid residue linked to the N-terminal end of the glycyl residue, said collagenase cutting said sequence specifically at the level of the X-glycyl linkage, and in that it is at least partly inhibited by myosine, also characterized in that it also contains collagen hydrolysate in the amount of 1 to 25 parts by weight, preferably 2 to 10 parts by weight of collagen hydrolysate per part by weight of collagenase.

8. Composition according to claim 7, characterized in that the collagenase is from cultures of Vibrio alginolyticus chemovar iophagus.

9. Pharmaceutical composition according to claim 8, characterized in that the collagenase is from the strain deposited at the C.N.C.M. under n° I-029.

10. Pharmaceutical composition according to claims 1 to 6, characterized in that it also contains a collagen hydrolysate in the amount of 1 to 25 parts by weight, preferably 2 to 10 parts by weight of collagen hydrolysate per part by weight of collagenase.

11. Pharmaceutical composition according to claim 10, characterized in that the collagenase is from cultures of Vibrio alginolyticus chemovar iophagus.

12. Pharmaceutical composition according to claim 10, characterized in that the collagenase is from the strain deposited at the C.N.C.M. under n° I-029.

13. Freeze dried and stabilized composition based on collagenase characterized in that it contains, in association with said collagenase, from 1 to 25 parts by weight, preferably 2 to 10 parts by weight of collagen hydrolysate per part by weight of collagenase.

14. Composition according to claim 13, characterized in that it contains a collagenase recognizing specifically a peptidic sequence X-glycyl-L-propyl, in which X is a natural amino acid residue linked to the N-terminal end of the glycyl residue, said collagenase cutting said sequence specifically at the level of the X-glycyl linkage and in association with said collagenase from 1 to 25 parts by weight, preferably 2 to 10 parts by weight of collagen hydrolysate per part by weight of collagenase.

15. Composition according to claim 13, characterized in that the collagenase it contains is from cultures of Vibrio alginolyticus chemovar iophagus.

16. Composition according to claim 13, characterized in that the collagenase is from the strain deposited at the C.N.C.M. under number I-029.

17. Freeze dried and stabilized composition based on three constituents — collagenase, proteases and endonucleases — according to claim 1, characterized in that it contains, 2 to 10 parts by weight of collagen hydrolysate per part by weight of collagenase.

18. Composition according to claim 17, characterized in that the three constituents are from cultures of Vibrio alginolyticus chemovar iophagus.

19. Composition according to claim 17, characterized in that the three constituents are from the strain deposited at the C.N.C.M. under number I-029.

20. Process for obtaining from an aqueous collagenase solution a freeze dried preparation of collagenase according to claims 13 to 19, without loss of collagenolytic activity, characterized in that the freeze drying of said solution is carried out in the presence of efficient proportions of collagen hydrolysate prealably introduced into said solution.

21. Process according to claim 20, characterized in that the proportions of collagen hydrolysate prealably introduced into the solution with respect to its content in collagenase are from 1 to 25 parts by weight, preferably 2 to 10 parts by weight of collagen hydrolysate with respect to one part by weight of collagenase.

22. Composition according to anyone of claims 1 to 19, under the form of a solution for the use in the treatment of dental caries or other alterations of the dental pulp in man or animal.

**Patentansprüche**

1. Pharmazeutische Zusammensetzung, insbesondere für die Verwendung bei der Behandlung von Erkrankungen, die sich in einer unkontrollierten Änderung der kollagenreichen Strukturen beim Menschen oder Tier manifestieren, wobei dei Zusammensetzung dadurch gekennzeichnet ist, daß sie in Assoziation mit einem pharmazeutischen Träger eine wirksame Dosis des Wirkstoffs mit den drei folgenden Bestandteilen in den entsprechenden relativen Proportionen enthält :

mindestens 0,350 μkat Kollagenase, die speziell eine Peptid-Sequenz X-Glycyl-L-Prolyl erkennt, bei der X ein Rest einer natürlichen Aminosäure ist, der an das N-terminale Ende des Glycylrestes gebunden ist, eine Sequenz, die sie speziell in Höhe der X-Glycyl-Bindung spaltet,

20-200 caseinolytische Einheiten, was den Gehalt an neutralen Proteasen angeht und

30-500 Nuklease-Einheiten, was die Endonukleasen angeht,

und daß sie darüber hinaus mindestens teilweise durch Myosin inhibiert ist.

2. Zusammensetzung nach Anspruch 1, dadurch gekennzeichnet, daß der Wirkstoff aus Kulturen von Vibrio alginolyticus chemovar iophagus stammt.

3. Zusammensetzung nach Anspruch 2, dadurch gekennzeichnet, daß der Wirkstoff aus dem bei dem C.N.C.M. unter der Nr. I-029 hinterlegten Stamm stammt.

4. Zusammensetzung nach einem der Ansprüche 1-3, dadurch gekennzeichnet, daß sie durch die Aktivität der Kollagenase ausgedrückte Dosen enthält, die, bezogen auf das Gesamtgewicht der Zusammensetzung, mindestens folgende Werte annehmen :

bis 0,05 μkat/g der pharmazeutischen Zusammensetzung·für Emulsionen, Salben, Pulver oder alle nicht wirklich flüssigen Kompositionen, in dem üblicherweise diesem Ausdruck gegebenen Sinn und

bis 0,05 μkat/ml der pharmazeutischen Zusammensetzung für die zur örtlichen Anwendung vorgesehenen Lösungen.

5. Zusammensetzung nach Anspruch 4, dadurch gekennzeichnet, daß sie von 0,1-2, vorzugweise 0,5-1 μkat Kollagenase pro g Emulsionen, Salben, Pulver oder nicht wirklich flüssige Zusammensetzungen oder 0,1-2, vorzugsweise 0,5-1 μkat Kollagenase pro ml Lösung aufweist.

6. Zusammensetzung nach den Ansprüchen 1-5, gekennzeichnet durch die relativen Anteile der oben genannten Bestandteile, die jeweils Aktivitäten entsprechen :

mindestens 0,350 μkat, was die Kollagenase angeht,

20-50 caseinolytische Einheiten, was die neutralen Proteasen angeht, und

30-60 Nuklease-Einheiten, was die Endonukleasen angeht.

7. Pharmazeutische Zusammensetzung, insbesondere für die Verwendung bei der Behandlung von Erkrankungen, die sich durch eine unkontrollierte Änderung von kollagenreichen Strukturen beim Menschen oder Tier manifestieren, dadurch gekennzeichnet, daß sie in Assoziation mit einem pharmazeutischen Träger eine wirksame Dose einer Kollagenase enthält, die speziell eine Peptid-Sequenz X-Glycyl-L-Prolyl erkennt, in der X ein Rest einer natürlichen Aminosäure ist, der an das N-terminale Ende des Glycylrestes gebunden ist, eine Sequenz, die sie speziell in Höhe der X-Glycyl-Bindung spaltet, und daß die Zusammensetzung darüber hinaus mindestens teilsweise durch das Myosin inhibiert ist, gekennzeichnet gleichermaßen durch die Tatsache, daß sie ebenfalls ein Kollagenhydrolysat im Verhältnis von 1-25 Gewichtsteilen, vorzugsweise 2-10 Gewichtsteilen Kollagenhydrolysat pro Gewichtsteil Kollagenase enthält.

8. Zusammensetzung nach Anspruch 7, dadurch gekennzeichnet, daß die Kollagenase aus Kulturen Vibrio alginolyticus chemovar iophagus stammt.

9. Pharmazeutische Zusammentzung nach Anspruch 8, dadurch gekennzeichnet, daß die Kollagenase aus dem bei dem C.N.C.M. unter der Nr. I-029 hinterlegten Stamm stammt.

10. Pharmazeutische Zusammensetzung nach den Ansprüchen 1-6, dadurch gekennzeichnet, daß sie ebenfalls ein Kollagenhydrolysat im Verhältnis von 1-25 Gewichtsteilen, vorzugsweise 2-10 Gewichtsteilen Kollagenhydrolysat pro Gewichtsteil Kollagenase enthält.

11. Pharmazeutische Zusammensetzung nach Anspruch 10, dadurch gekennzeichnet, daß die Kollagenase aus Kulturen von Vibrio alginolyticus chemovar iophagus stammt.

12. Pharmazeutische Zusammensetzung nach Anspruch 10, dadurch gekennzeichnet, daß die Kollagenase aus dem bei dem C.N.C.M. unter der Nr. I-029 hinterlegten Stamm stammt.

13. Lyophilisierte und stabilisierte Zusammensetzung auf Basis einer Kollagenase, dadurch gekennzeichnet, daß sie in Verbindung mit der Kollagenase 1-25 Gewichtsteile, vorzugsweise 2-10 Gewichtsanteile Kollagenhydrolysat pro Gewichtsteil Kollagenase enthält.

14. Zusammensetzung nach Anspruch 13, dadurch gekennzeichnet, daß sie eine Kollagenase enthält, die speziell eine Peptid-Sequenz X-Glycyl-L-Prolyl erkennt, in der X ein Rest einer natürlichen Aminosäure ist, der an das N-terminale Ende des Glycylrests gebunden ist, eine Sequenz, die sie speziell in Höhe der X-Glycyl-Bindung spaltet, und daß die Zusammensetzung in Verbindung mit der Kollagenase 1-25 Gewichtsteile, vorzugsweise 2-10 Gewichtsteile Kollagenhydrolysat pro Gewichtsteil Kollagenase enthält.

15. Zusammensetzung nach Anspruch 13, dadurch gekennzeichnet, daß die Kollagenase, die sie enthält, von Vibrio alginolyticus chemovar iophagus-Kulturen stammt.

16. Zusammensetzung nach Anspruch 13, dadurch gekennzeichnet, daß die Kollagenase aus dem bei dem C.N.C.M. unter der Nr. I-029 hinterlegten Stamm stammt.

17. Lyophilisierte und stabilisierte Zusammensetzung auf Basis von drei Bestandteilen — Kollagenase, Proteasen und Endonukleasen — nach Anspruch 1, dadurch gekennzeichnet, daß sie 2-10 Gewichtsteile Kollagenhydrolysat pro Gewichtsteil Kollagenase enthält.

18. Zusammensetzung nach Anspruch 17, dadurch gekennzeichnet, daß die drei Bestandteile von Kulturen des Vibrio alginolyticus chemovar iophagus stammen.

19. Zusammensetzung nach Anspruch 17, dadurch gekennzeichnet, daß die drei Bestandteile von dem bei dem C.N.C.M. unter der Nr. I-029 hinterlegten Stamm stammen.

20. Verfahren zur Herstellung einer lyophilisierten Kollagenasezubereitung ausgehend von einer wässrigen Kollagenase-Lösung, nach den Ansprüchen 13-19, ohne Verlust an kollagenolytischer Aktivität, gekennzeichnet durch die Tatsache, daß die Lyophilisierung der genannten Lösung im Beisein von wirksamen Anteilen von Kollagenhydrolysat durchgeführt wird, das vorher in die Lösung eingeführt wurde.

21. Verfahren nach Anspruch 20, dadurch gekennzeichnet, daß die Anteile des vorher in die Lösung eingeführten Kollagenhydrolysats gegenüber seinem Anteil an Kollagenase von 1 bis 25 Gewichtsteile, vorzugsweise 2-10 Gewichtsteile Kollagenhydrolysat gegenüber einem Gewichtsteil Kollagenase betragen.

22. Zusammensetzung nach einem der Ansprüche 1-19, in Form einer Lösung zur Verwendung bei der Behandlung von Zahnkaries oder anderen Änderungen der Zahnpulpa beim Menschen oder Tier.

0 115 974

FIG.1.

FIG.2.